# EUROPÄISCHE PATENTSCHRIFT

(11) **EP 2 725 019 B1**
(45) Veröffentlichungstag und Bekanntmachung des Hinweises auf die Patenterteilung: **08.04.2015**
(21) Anmeldenummer: 12190199.5
(22) Anmeldetag: 26.10.2012
(51) Int. Cl.: C07D 319/12

(54) **Verfahren zur Herstellung cyclischer Diester, insbesondere Dilactid**
Method for the preparation of cyclic diesters, in particular dilactide
Procédé de fabrication de diesters cycliques, notamment de dilactide

(43) Veröffentlichungstag der Anmeldung: 30.04.2014
(73) Patentinhaber: Uhde Inventa-Fischer GmbH, 13509 Berlin (DE)
(72) Erfinder: Dr. Paetz, Caspar, 13353 Berlin (DE); Mühlbauer, Udo, 10119 Berlin (DE); Dr. Driouch, Habib, 04347 Leipzig (DE)
(74) Vertreter: Pfenning, Meinig & Partner GbR

(56) Entgegenhaltungen:
- WO-A1-2011/104728
- DATABASE CAPLUS [Online] CHEMICAL ABSTRACTS SERVICE, COLUMBUS, OHIO, US; 24. Dezember 2010 (2010-12-24), XP002693836, Database accession no. 2010:1551333 & CN 101 906 041 A (XIAOGAN YISHENG NEW MATERIALS CO LTD) 8. Dezember 2010 (2010-12-08)

## Beschreibung

Die vorliegende Erfindung betrifft ein Verfahren zur Herstellung von cyclischen Carbonsäureestern, insbesondere intramolekularen Lactonen wie z. B. Dilactid, wobei die Herstellung dieser cyclischen Ester aus oligomeren Carbonsäuren durch cyclisierende Depolymerisation erfolgt. Bei dieser Reaktion fällt als Nebenprodukt ein Kondensationsprodukt dieser oligomeren Carbonsäuren an, d. h. ein Gemisch höhermolekularer oligomerer Carbonsäuren, die in einem weiteren Schritt hydrolisiert und somit rückgewonnen werden. Dieses Hydrolysat kann erneut der in der ersten Stufe durchgeführten cyclisierenden Depolymerisation zugesetzt werden.

Alle derzeit gängigen Verfahren für die Herstellung von PLA im großtechnischen Maßstab basieren auf der Ringöffnungspolymerisation von Dilactid. Die Herstellung des Dilactids erfolgt dafür durch die cyclisierende Depolymerisation von Milchsäure-Polykondensat. Dabei wird ein Milchsäure-Polykondensat mit niedrigem mittlerem Molekulargewicht (500...2000 Da) unter Zugabe eines Katalysators bei niedrigem Druck erhitzt, wodurch sich das Dilactid bildet und dampfförmig abgezogen wird. Dabei bleibt zwangsweise ein Rückstand zurück, welcher aus dem Katalysator, einem Milchsäure-Polykondensat mit einem mittleren Molekulargewicht von 3000...5000 Da und verschiedenen Verunreinigungen besteht. Die Menge an Rückstand hat unmittelbaren Einfluss auf die Gesamtausbeute des Verfahrens. Es besteht daher die Tendenz, das Milchsäure-Polykondensat möglichst vollständig zu Dilactid umzusetzen, um die Ausbeute zu erhöhen.

Eine Besonderheit der PLA-Herstellung liegt darin begründet, dass Milchsäure in zwei optisch aktiven Formen, der S(+)- und der R(-)-Form, vorliegt welche auch als L- und D-Milchsäure bezeichnet werden. Bei der Herstellung von Dilactid können sich demnach drei optisch verschiedene Formen bilden, das L-Lactid (mit absoluter S,S-Konfiguration der Stereozentren), das Meso-Lactid (mit absoluter R,S-Konfiguration der Stereozentren) und das D-Lactid (mit absoluter R,R-Konfiguration der Stereozentren). Gewünscht ist vorrangig L-Lactid, welches auch vorwiegend gebildet wird, da zumeist die eingesetzte Milchsäure zu >99% L-Isomere enthält.

Es kann allerdings grundsätzlich in allen Stufen des Verfahrens zur Umwandlung von L-Milchsäure in D-Milchsäure beziehungsweise von L-Lactid in Meso-Lactid und D-Lactid kommen (Racemisierung). Nachteilig für die Gesamtausbeute des Verfahrens ist, dass bei Umsätzen über die üblichen 95% hinaus der Grad der Racemisierung von L-Lactid zu Meso-Lactid stark ansteigt. Somit wird die zusätzliche Ausbeute auf Kosten eines hochwertigen Produkts erreicht, da nur L-Lactid mit einem Anteil von maximal 10...12% Meso-Lactid zu einem Polymer mit hohem Schmelzpunkt polymerisiert werden kann.

Im bisherigen Stand der Technik sind umfangreiche Informationen zur Hydrolyse von PLA bzw. Milchsäure-Polykondensat (auch: Milchsäure-Oligomere) vorhanden. Dazu gehören z.B. WO2011/029648, WO2010/118954, US5229528, EP0628533, oder EP0893462. Es sind ebenfalls Möglichkeiten zur weiteren Erhöhung des Umsatzes publiziert, z.B. DE19637404 oder US7557224. Dabei wird bei erhöhten Temperaturen unter Zugabe eines Racemisierungs-Inhibitors der Rückstand möglichst weit reduziert, ohne die Racemisierung zu sehr zu erhöhen. Im bisherigen Stand der Technik sind auch umfangreiche Informationen bezüglich der Reinigung von Milchsäure und der Rückgewinnung von Lactid vorhanden: DE 19637 404 A1, DE 60317581 T2, DE 69405201 T2 (EP 0657447 Bl), DE 69815369 T2, EP 1276890 B1, US 2011/0155557 A1. Bei der Polymerisation von Milchsäure sind ein oder mehrere Katalysatoren bekannt, die dem Polymerisationsreaktor zugeführt werden. Das kann Metalle oder anorganische /organische Metall Verbindungen, wie z. B. Sn-, Zn- oder Fe-Verbindungen, umfassen (DE 19637 404 A1, DE 60317581 T2, DE 60317581 T2).

Es existiert jedoch keine bekannte Quelle, bei der die Verringerung der Racemisierung während der Depolymerisation mit der Hydrolyse des Rückstands und anschließender Rückführung in den Prozess beschrieben ist. Auch werden keine Angaben über Katalysatorentfernung oder -Rückgewinnung beschrieben.

WO2011/029648 und WO2010/118954 (Galactic): Darin wird die Nutzung von Ethyllactat als Hydrolysemedium für die Zersetzung von PLA beschrieben. Nachteilig ist die Entstehung von Ethanol, welches im Anschluss an die Hydrolyse abdestilliert werden muss.

WO2010/118955 (Galactic): Darin wird die Alkoholyse mit verschiedenen Lactaten als Hydrolysemedium beschrieben, auch hier muss der jeweilige Alkohol später entfernt werden.

US2526554: Frühe Erwähnung der Alkoholyse. Allerdings nur für Polyester aus beta-Lactonen.

US5229528: Hydrolyse von PHA (wozu auch PLA gehört) mit Wasser bei erhöhtem Druck und erhöhter Temperatur. Nachteilig ist die Hydrolyse im geschlossenen Gefäß, außerdem ist das Verfahren diskontinuierlich. Es werden außerdem sehr lange Reaktionszeiten gebraucht, da reines Wasser als Hydrolysemedium genutzt wird.

EP0628533 (DuPont): Beschreibung der Hydrolyse durch Wasser, Alkohole, Amine, Diamine und Mischungen daraus. Beinhaltet eine Abtrennung des Hydrolysats im 1. Anspruch.

EP0893462 (Cargill): In diesem Patent (Beispiel 4) ist die Hydrolyse der Depolymerisationsrückstände mit 88%iger Milchsäure beschrieben. Nachteilig ist dabei, dass keine Katalysatorentfernung stattfindet und dass sich damit durch dessen Anreicherung ein stetig zunehmender Anteil an D-Isomeren einstellen kann.

Faisal 2007 (Asian Journal of Chemistry Vol. 19, No. 3,1714-1722): Dort wird die Hydrolyse von PLA bei hohen Temperaturen (> 160 °C) beschrieben. Nachteilig ist dabei eben dieses hohe Temperaturniveau, weil dabei verstärkt Racemisierung auftreten kann.

Yagihashi 2010 (Ind. Eng. Chem. Res., 2010, 49 (3), pp 1247-1251): Dort benutzt man verdünnte Natronlauge im Temperaturbereich von 70-180 °C. Keine Racemisierung innerhalb von 20-60 min Reaktionszeit. Nachteilig ist die Notwendigkeit zur anschließenden Entfernung des Natriums.

US7557224 (Kyushu Institute of Technology): Beschrieben ist die selektive Pyrolyse von Polykondensat unter Zugabe von Aluminium-Hydroxid. Nachteilig ist die extrem hohe Temperatur von über 300 °C.

DE 19637404: Beschrieben ist die direkte Rückgewinnung von Lactid aus PLA, allerdings geht dies nur mit massivem Katalysatoreinsatz und bei sehr hohen Temperaturen.

Fan 2003 (Green Chemistry, 2003, 5, 575-579): Pyrolyse des Depolymerisations-Rückstandes bei 250...300 °C unter Zugabe von Calzium-Komponenten zur Verhinderung einer übermäßigen Racemisierung.

DE 19637 404 A1 (Schimadzu Corp): Darin wird ein Verfahren zur Rückgewinnung von Lactid aus Polymilchsäure (PLA), umfassend die Hitzebehandlung einer PLA, beschrieben.

DE 60317581 T2 (Tate & Lyle p.lc.): Das Patent betrifft ein Verfahren zur Reinigung von Dilactid aus einem dampfförmigen Rohdilactid-Produkstrom, der mindestens das Dilactid, Milchsäure, Wasser und lineare Milchsäure-Oilomere umfasst.

DE 69405201 T2 (EP 0657447 B1) (Musashino Chemical Laboratory Ltd): Darin wird ein Verfahren zur Reinigung von Dilactid beschrieben.

DE 69815369 T2 (Cargill): Die Erfindung betrifft die Verarbeitung von Milchsäure, insbesondere die Trennung von Milchsäureströmen und Lactidsalzströmen von Mischungen, die Isolierung und Verarbeitung der Milchsäure und die Isolierung des Lactatslazes in bevorzugten Formen.

EP 1276890 B1 (Uhde Inventa-Fischer GmbH): Die Erfindung betrifft ein Verfahren zur Herstellung von Polymilchsäure (PLA) aus fermentativ hergestellter Milchsäure wobei Stärke als Rohstoff verwendet wird.

US 2011/0155557 A1 (Futerro): Die Erfindung betrifft ein Verfahren zur Herstellung von Lactid aus Milchsäure.

Im Rahmen der vorliegenden Anmeldung werden folgende Definitionen verwendet:
Carboxylendgruppen des PLA-Hydrolysats, Molmasse der PLA-Oligomeren: Das PLA-Oligomer wird in Aceton gelöst. Nach Zugabe von Methanol wird die Lösung mit 0,1 N benzylalkoholischer KOH-Lösung titriert. Der Endpunkt wird potentiometrisch erfasst. Aus der Carboxylendgruppenkonzentration ("COOH"), gemessen in mmol/kg, kann das Zahlenmittel der Molmasse berechnet werden nach der Gleichung Mn =10⁶/COOH.
Carboxylgruppen im Lactid: Die Dilactidprobe wird in Methanol gelöst und anschließend auf die gleiche Weise titriert wie bei der Carboxylendgruppenbestimmung im PLA-Oligomer.
Optische Isomere des Dilactids: Die Dilactid-Probe wird in einem Gemisch aus 90/10 ml/ml n-Hexan/Ethanol gelöst. Die gelösten Komponenten werden mit HPLC auf einer chiralen Säule getrennt und mit einem UV-Detektor bei 223 nm analysiert.
D-Anteil in Milchsäure und PLA: Eine Probe aus PLA oder einem PLA-Oligomer wird mit 1-N Natronlauge am Rückfluss siedend hydrolysiert und nach Abkühlen neutralisiert. Die neutralisierte Probe wird mit 3-millimolarer Kupfersulfatlösung im Verhältnis 1/9 ml/ml vermischt und mit HPLC auf einer stereospezifischen Säule in die Komponenten getrennt, die anschließend mit einem UV-Detektor bei einer Wellenlänge von 238 nm analysiert werden. Eine Milchsäureprobe wird direkt in der 3-millimolaren Kupfersulfatlösung gelöst und wie beschrieben mit HPLC analysiert.
Dilactid: zyklischer Ester aus 2 Milchsäuremolekülen, der in der Form des reinen L-Lactids (S,S-Lactid), D-Lactids (R,R-Lactids) oder meso-Lactids (S,R-Lactid) vorkommen kann oder (in den meisten Fällen) in der Form eines Gemisches aus mindestens zwei dieser Komponenten. Die zyklisierende Depolymerisation erzeugt Roh-Dilactid, das neben den genannten Isomeren des Lactids noch lineare Oligomere, höhere zyklische Oligomere und Reste von Milchsäure und Wasser enthalten kann. Unter L-Lactid ist der zyklische Ester aus zwei L-Milchsäureeinheiten zu verstehen, unter D-Lactid der zyklische Ester aus zwei D-Milchsäureeinheiten, unter meso-Lactid der zyklische Ester aus einer D- und einer L-Milchsäureeinheit. Racemisches Lactid (rac-Lactid) ist ein 1:1-Gemisch aus L- und D-Lactid.

Der Begriff der Racemisierung bezeichnet in diesem Zusammenhang die Umwandlung von L- in D-Einheiten und umgekehrt. Da bei diesem Prozess aber von L-Milchsäure ausgegangen wird, ist erfindungsgemäß mit einer zunehmendem Racemisierung immer die vermehrte Bildung von D-Einheiten gemeint. Aufgrund der unterschiedlichen Stabilität der chiralen Zentren von Milchsäure und Dilactid erfolgt diese Racemisierung beim Dilactid deutlich einfacher.
Zyklisierende Depolymerisation: Diese Reaktion ist die Umkehrreaktion der Ringöffnungspolymerisation, die in fast allen industriellen Prozessen zum Aufbau des PLA aus Lactid verwendet wird. Sie ist am fertigen PLA unerwünscht, weil sie während der Verarbeitung zum Molmassenabbau und damit zur Verschlechterung der Produkteigenschaften führt. Bei der Herstellung von Dilactid wird sie genutzt, um aus dem durch Polykondensation der Milchsäure erhaltenen Oligomer ein Roh-Dilactid herzustellen, das nach Reinigung den Ausgangsstoff für die Ringöffnungspolymerisation bildet. Roh-Dilactid enthält neben 80...90% Dilactid noch Reste von Wasser, Milchsäure, kurzkettige Milchsäure-Oligomere sowie Spuren sonstiger Verunreinigungen (z.B. organische Säuren).

Die theoretisch maximal mögliche Ausbeute bei diesem Prozess beträgt 80%, das heißt aus 1,25 kg Milchsäure wird 1 kg Dilactid gebildet. Die erfindungsgemäß angegebenen Ausbeuten beziehen sich auf diesen maximal möglichen Zustand. Eine Gesamtausbeute von 95% bezeichnet also einen Prozess, bei dem aus 1,25 kg Milchsäure 0,95 kg Dilactid hergestellt wurden. Da die durch Verdampfung in die Roh-Dilactid-Fraktion überführte Milchsäure zu einem späteren Zeitpunkt des Verfahrens abgetrennt und zurückgeführt werden kann, ist so erfindungsgemäß mit einem Roh-Dilactid, welches 80% Dilactid enthält, eine Gesamtausbeute von 95% und mehr erreichbar.
Polylactid (PLA): Polymer aus Milchsäureeinheiten, z.B. hergestellt durch ringöffnende Polymerisation des L-, D- oder meso-Lactids oder einer Mischung aus zwei oder drei dieser Lactide. Es kann sich auch um eine Mischung aus Polymeren aus den genannten reinen oder gemischten Lactiden handeln. PLA weist in der Regel ein zahlengemitteltes Molekulargewicht > 10.000 g/mol auf. Die Qualität des PLAs, das aus Dilactid hergestellt wird, hängt ab von dessen Gehalt an D-Lactid und meso-Lactid-Einheiten. Mit steigendem Gehalt an D-Milchsäure-Einheiten im vorwiegend aus L-Lactid-Einheiten aufgebauten PLA sinken dessen Schmelzepunkt, Wärmeformbeständigkeit, Kristallisationsgeschwindigkeit sowie Kristallisationsgrad. Wenn dieser Gehalt an D,D-Einheiten im PLA 6% (entspricht 12% Meso-Lactid da dieses nur eine D-Einheit beinhaltet) übersteigt, ist das Polymer amorph, der Schmelzpunkt fällt mit der Glasübergangstemperatur von z.B. 55°C zusammen. Gleiches gilt für meso-Lactid und L-Lactid in einem vorwiegend aus D-Einheiten aufgebauten PLA.
Hydrolysierendes Medium: Erfindungsgemäß sind darunter Wasser, Milchsäure oder ein Gemisch aus Wasser und Milchsäure zu verstehen. Reine Milchsäure bewirkt mit PLA zwar keine Hydrolyse, sondern eine Umesterung. Die Wirkung - der Abbau zu PLA-Oligomeren - ist jedoch dieselbe, wie bei der Hydrolyse. Eine Unterscheidung erübrigt sich deshalb für die Zwecke dieser Erfindung. Milchsäure enthält in industriell verfügbaren Qualitäten jedoch stets Wasser und fördert die Hydrolyse katalytisch. Der erfindungsgemäß verwendete Begriff "Milchsäure" schließt somit geringe Anteile von Wasser ein. Auch andere wasserhaltige Säuren fördern die Hydrolyse, müssen jedoch anschließend aus dem Produkt entfernt werden, da sie anders als Milchsäure die Depolymerisation zum Lactid oder dessen Polymerisation zu PLA stören.
Partielle Hydrolyse: Wie alle Polyester ist auch PLA der hydrolytischen Spaltung der Polymerketten zugänglich. Was bei der Polymerisation und Verarbeitung vermieden werden muss, wird im erfindungsgemäßen Verfahren dazu benutzt, das Polymer gezielt bis auf eine gewünschte Molmasse abzubauen. Obwohl die Hydrolyse des PLA bereits bei Temperaturen unterhalb des Schmelzpunkts abläuft, sind die dafür benötigten Verweilzeiten oder die benötigte Wasserkonzentration im Polymer sehr hoch. Ein technischer Prozess ist dann vorteilhaft, wenn er in kurzen Verweilzeiten zum Ziel führt, was zu geringen Baugrößen bei den Apparaten führt. Der erfindungsgemäße Prozess benutzt darüber hinaus die geringste mögliche Wassermenge zur Hydrolyse, mit dem Ziel, nur genau die gewünschte Molmasse zu erreichen. Damit wird überschüssiges Wasser vermieden, das nach Abschluss der Hydrolysereaktion mit Aufwand an Energie und Apparaten wieder entfernt werden muss. Eine Hydrolyse mit deutlich größerer Wassermenge führt darüber hinaus zur stärkeren partiellen Racemisierung (JP 2009-249508; M. Faisal et al, Asian Journal of Chemistry Vol. 19, No. 3 (2007), S. 1714).
Sn-Fällung, Filtration/ Zentrifugation und H₂S-Entfernung: In einem ersten Schritt der Aufbereitung des Hydrolysats wird der Zinn-Katalysator aus dem Hydrolysat durch Zugabe von Fällungsmittel bei Raumtemperatur unter leichtem Rühren ausgefällt. Der Niederschlag wird durch Filtration entfernt. Das Filtrat enthält anschließend nur noch wenige ppm Zinn. Bei der Hydrolysat-Behandlung mit Fällungsmittel entsteht Schwefelwasserstoff (H₂S). Diese kann durch eine Erhöhung der Temperatur auf 40 °C unter Rühren entfernt werden. Das ausgefällt Zinn kann für die Synthese von Sn-Oxid genutzt werden und dann als Katalysator für die Dilactid-Polymerisation wiederverwendet werden.
Nanofiltration: Nach der Sn-Fällung und der Sn-/ H₂S-Entfernung wird anschließend die nun zinnarme Lösung einer Nano-Filtration (NF) zugeführt. Das ist ein druckgetriebenes Membranverfahren, welches Partikeln oder Ionen im Nanometer-Bereich (ca. 1-10 nm) wie Schwermetall-Ionen zurückhält. Weiterhin können auch Farbstoffe durch die NF zurückgehalten werden. An die Nanofiltration (NF) schließt sich das Polishing, die Endreinigung, der Milchsäure an. Hier ist es das Ziel, die Milchsäure von allen verbleibenden Verunreinigungen einschließlich Farbstoffen zu befreien. Während des Polishings (Ionenaustausch erste Stufe und zweite Stufe) wird die Milchsäure schrittweise durch Eindampfung aufkonzentriert. Am Ende der Reinigungsstufe sollte die LA Polymer-Qualität erreicht haben, sodass sie der Polymerisation zugeführt werden kann.
Ionenaustausch: Die Verwendung von Ionenaustauschern ist ein wesentlicher Bestandteil der Aufbereitung des Hydrolysats und wird als Polishing bezeichnet. Ionenaustauscher, wie sie heute verwendet werden, sind in Wasser nicht lösliche aber quellbare Kunstharze mit substituierten ionenaktiven Gruppen. Damit stellen sie Elektrolyte in fester Form dar. Ionen bestimmter Art können aus der wässrigen Phase in die wasserhaltige Austauscherphase diffundieren und werden an den austauschaktiven Gruppen gegen gleichgeladene Ionen in äquivalenter Menge ausgetauscht.

Aufgrund der beschriebenen hohen Bedeutung von Monomeren mit möglichst hoher optischer Reinheit für die Herstellung von Polylactiden, ist es somit Aufgabe der vorliegenden Erfindung, ein Verfahren zur Herstellung von cyclischen Estern, insbesondere Dilactid anzugeben, mit dem sich das Dilactid in einer möglichst hohen Ausbeute und in möglichst hoher optischer Reinheit erhalten lässt.

Diese Aufgabe wird mit den Merkmalen des Patentanspruchs 1 gelöst. Die abhängigen Patentansprüche stellen dabei vorteilhafte Weiterbildungen dar.

Gemäß der vorliegenden Erfindung wird somit ein Verfahren zur Herstellung von cyclischen Estern der allgemeinen Formel I 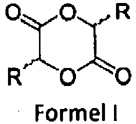 bereitgestellt, bei dem
a) in einer ersten Stufe ein Teil eines Gemischs oligomerer Carbonsäuren der Formel II 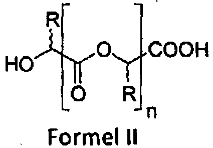 wobei in den Formeln I bis II jeweils R ausgewählt ist aus linearen oder verzweigten aliphatischen Resten mit 1 bis 6 Kohlenstoffatomen und in Formel II der gemittelte Wert für n zwischen 2 und 40 liegt,
   durch cyclisierende Depolymerisation in den cyclischen Diester der allgemeinen Formel I überführt und der dabei entstehende cyclische Diester der allgemeinen Formel I abgetrennt wird, wobei der nicht in den cyclischen Diester der allgemeinen Formel I überführte Teil der oligomeren Carbonsäure der Formel II während der cyclisierenden Depolymerisation zu einem Rückstand abreagiert, der ein Gemisch oligomerer Carbonsäuren der Formel II mit einem höheren gemittelten Wert für n als das in die erste Stufe aufgegebene Gemisch oligomerer Carbonsäuren der Formel II beinhaltet,
b) in einer zweiten Stufe der in der ersten Stufe erhaltene Rückstand zu einem Gemisch oligomerer Carbonsäuren der Formel II, wobei der gemittelte Wert für n zwischen 1 und 20 liegt, zu einer alpha-Hydroxycarbonsäure der Formel III 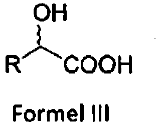 oder einem Gemisch aus oligomeren Carbonsäuren der Formel II mit einem gemittelten Wert für n zwischen 1 und 20 und einer alpha-Hydroxycarbonsäure der Formel III hydrolysiert wird, und
   das in der zweiten Stufe erhaltene Hydrolysat zumindest teilweise oder vollständig in die erste Stufe aufgegeben wird.

Erfindungsgemäß ist nunmehr vorgesehen, den Depolymerisationsumsatz in der ersten Stufe zu limitieren, d.h. die cyclisierende Depolymerisation der ersten Stufe wird nur solange durchgeführt, bis maximal 98 Gew.-% des eingesetzten Gemischs oligomerer Carbonsäuren der Formel II durch die cyclisierende Depolymerisation in den cyclischen Diester der allgemeinen Formel I überführt sind.

Überraschenderweise konnte festgestellt werden, dass mit einer derartigen Verfahrensweise die Ausbeute eines Verfahrens zur Herstellung von cyclischen Diestern deutlich erhöht werden kann, ohne dabei die Racemisierungsrate zu erhöhen und damit die erreichbare Produktqualität zu verringern. Mit dem erfindungsgemäßen Verfahren sind Gesamtausbeuten von bis zu > 99 % bei gleichzeitig akzeptabler Racemisierung möglich. Die wesentliche Idee ist dabei, dass eine neue Verfahrensweise für den Schritt der Depolymerisation mit einem Aufbereitungsverfahren für den Rückstand kombiniert und der Umsatz in der ersten Stufe begrenzt wird. Durch die veränderte Fahrweise der Depolymerisation wird die Racemisierung verringert, gleichzeitig aber die Rückstandsmenge vergrößert. Durch die Rückführung des Rückstands in einen vorherigen Prozessschritt nach Aufarbeitung durch Hydrolyse und gegebenenfalls notwendige Reinigungsschritte (Filtration, Ausfällung, Ionenaustausch) wird die Gesamtausbeute des Prozesses deutlich erhöht, wobei ggf. der dabei erreichte Grad an Racemisierung maximal so hoch ist, wie ohne diesen Verfahrensschritt, tendenziell sogar niedriger.

Gemäß einer bevorzugten Ausführungsform des erfindungsgemäßen Verfahrens wird das in die erste Stufe aufgegebene Gemisch oligomerer Carbonsäuren der Formel II durch eine vorgeschaltete Polykondensation einer alpha-Hydroxycarbonsäure der Formel III 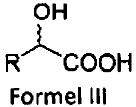 erhalten, wobei R die oben angegebene Bedeutung aufweist. Gemäß dieser vorteilhaften Variante wird das für die erste Stufe des erfindungsgemäßen Verfahrens zur Erzeugung von cyclischen Estern eingesetzte Gemisch oligomerer Carbonsäuren der Formel II dabei direkt durch Polykondensation der entsprechenden alpha-Hydroxycarbonsäure der Formel III erzeugt. Diese Polykondensation der alpha-Hydroxycarbonsäuren der Formel III kann dabei unmittelbar beispielsweise in situ in einem vorgelagerten Reaktor erzeugt werden, das dabei entstehende Gemisch oligomerer Carbonsäuren der Formel II kann direkt für die weitere cyclisierende Depolymerisation eingesetzt werden. Es ist somit keine Abtrennung bzw. Aufarbeitung der in dieser Verfahrensvariante entstehenden oligomeren Carbonsäuren der Formel II notwendig.

Weiter ist es bevorzugt, wenn alternativ oder zusätzlich zur Aufgabe des Hydrolysats in die erste Stufe das Hydrolysat teilweise oder vollständig in die vorgeschaltete Polykondensation der alpha-Hydroxycarbonsäure der Formel III aufgegeben wird. Gemäß dieser Verfahrensvariante kann das in der zweiten Stufe des erfindungsgemäßen Verfahrens anfallende Hydrolysat unmittelbar auch in die vorgeschaltete Polykondensationsstufe der alpha-Hydroxycarbonsäure der Formel III aufgegeben werden. Für den Fall, dass das Hydrolysat eine alpha-Hydroxycarbonsäure der Formel III enthält, kann diese dort erneut zu oligomeren Carbonsäuren kondensiert werden, ggf. enthaltene niedermolekulare oligomere Carbonsäuren der Formel II können beispielsweise weiter kondensiert werden. In jedem Falle zeichnet sich eine derartige Verfahrensvariante durch eine hohe stoffliche Ökonomie der eingesetzten Edukte auf, da nicht in den cyclischen Ester gemäß der Formel I überführte Reaktionsbestandteile in einem Kreislauf wiederverwertet werden können, indem sie erneut in die entsprechenden Reaktionsstufen aufgegeben werden.

Insbesondere ist es vorteilhaft, wenn die cyclisierende Depolymerisation der ersten Stufe solange durchgeführt wird, bis maximal 95 Gew.-%, bevorzugt von 50 bis 95 Gew.-%, besonders bevorzugt von 60 bis 85 Gew.-% des eingesetzten Gemischs oligomerer Carbonsäuren der Formel II durch die cyclisierende Depolymerisation in den cyclischen Diester der allgemeinen Formel I überführt sind. Durch eine derartige Verfahrensführung kann bei der cyclisierenden Depolymerisation eine Racemisierung weitestgehend vermieden werden, d. h. eine Änderung der absoluten Stereokonfiguration der jeweiligen Stereozentren in den oligomeren Carbonsäuren der Formel II stattfindet. Durch die Hydrolyse und Rückführung des nicht umgesetzten Rückstandes wird zudem die Gesamtausbeute dieses Verfahrens nicht beeinträchtigt, so dass eine äußerst vorteilhafte ökonomische Verfahrensweise resultiert.

Eine weitere vorteilhafte Ausführungsform des erfindungsgemäßen Verfahrens sieht vor, dass die in der zweiten Stufe durchgeführte partielle Hydrolyse des Rückstandes durch Umsetzung des Rückstandes mit einem hydrolysierenden Medium, ausgewählt aus der Gruppe bestehend aus Wasser, einer alpha-Hydroxycarbonsäure der Formel III oder Gemischen aus Wasser und einer alpha-Hydroxycarbonsäure der Formel III mit einem bevorzugten Wassergehalt von 1 bis 99 Gew.-%, bevorzugt 10 bis 80 %, besonders bevorzugt 20 bis 60 % durchgeführt wird.

Eine weitere bevorzugte Variante sieht vor, dass die in der zweiten Stufe durchgeführte partielle Hydrolyse bei Drücken zwischen 500 mbar und 2 bar, bevorzugt bei 900 mbar bis 1100 mbar und/oder bei Temperaturen zwischen 50 und 300 °C, bevorzugt zwischen 80 und 120 °C durchgeführt wird.

Zudem ist es bevorzugt, eine Reinigung des Hydrolysats vor Rückführung in die erste Stufe und/oder die vor der ersten Stufe vorgelagerte Polykondensation durchzuführen. Insbesondere erfolgt eine derartige Reinigung durch Filtration, Nanofiltration, Zentrifugation, Destillation, Ausfällung und/oder Abtrennung eines ggfs. vorhandenen Katalysators, Ionentauschverfahren oder mehrerer der zuvor genannten Reinigungsverfahren.

Die cyclisierende Depolymerisation in der ersten Stufe kann dadurch beschleunigt werden, dass in der ersten Stufe ein Katalysator, bevorzugt ein Zinn-enthaltender Katalysator, insbesondere Zinn-(IV)-Octoat eingesetzt wird.

Bevorzugte Temperaturen bei der in der ersten Stufe durchgeführten cyclischen Depolymerisation liegen dabei zwischen 100 und 300 °C, bevorzugt zwischen 150 und 250 °C.

Weiter ist es vorteilhaft, wenn die cyclisierende Depolymerisation und/oder Abtrennung des in der ersten Stufe entstehenden cyclischen Diesters der allgemeinen Formel I bei gegenüber Normalbedingungen reduzierten Drücken, bevorzugt bei Drücken zwischen 0,1 und 500 mbar, weiter bevorzugt zwischen 10 und 100 mbar durchgeführt wird.

Insbesondere eignet sich das vorliegende Verfahren zur Herstellung von optisch aktiven cyclischen Estern der allgemeinen Formel I, d. h. cyclischen Estern der Formel I, bei denen R lineare oder verzweigte aliphatische Reste mit 1 bis 6 Kohlenstoffatomen darstellt. Besonders bevorzugt hierbei ist, wenn R = Methyl, d. h. der cyclische Diester gemäß der Formel I Dilactid ist.

Hierbei ist es insbesondere bevorzugt, wenn die entsprechenden Diester eine stereoisomere Reinheit von mind. 95 %, bevorzugt mind. 98 %, insbesondere mind. 99 % aufweisen, d. h. die Stereozentren in Formel II zu mindestens 95 %, bevorzugt zu mindestens 98 %, besonders bevorzugt zu mindestens 99 % *S-*Konfiguration oder *R*-Konfiguration besitzen.

Für den Fall, dass die Stereozentren in Formel II zu mindestens 95 %, bevorzugt zu mindestens 98 %, besonders bevorzugt zu mindestens 99 % *S-*Konfiguration besitzen, ist es bevorzugt, wenn die cyclisierende Depolymerisation der ersten Stufe solange durchgeführt wird, bis maximal 10 %, bevorzugt maximal 5 %, besonders bevorzugt maximal 3 % des abgetrennten cyclischen Diesters der allgemeinen Formel I *R*,*S*-Konfiguration und/oder *R,R*-Konfiguration aufweisen.

Alternativ ist es bevorzugt, dass wenn die Stereozentren in Formel II zu mindestens 95 %, bevorzugt zu mindestens 98 %, besonders bevorzugt zu mindestens 99 % *R*-Konfiguration besitzen, die cyclisierende Depolymerisation der ersten Stufe solange durchgeführt wird, bis maximal 10 %, bevorzugt maximal 5 %, besonders bevorzugt maximal 3 % des abgetrennten cyclischen Diesters der allgemeinen Formel I *R*,*S*-Konfiguration und/oder *S*,*S*-Konfiguration aufweisen.

Gemäß den zuvor genannten bevorzugten Ausführungsformen ist es somit vorteilhaft, die cyclisierende Depolymerisation der ersten Stufe so zu führen, dass eine möglichst geringe Racemisierung statt findet. Die stereoisomere Reinheit des erhaltenen und abgetrennten cyclischen Diesters kann beispielsweise über chirale HPLC-Chromatografie auf an und für sich aus dem Stand der Technik bekannte Art und Weise festgestellt werden.

Alternativ zu den zuvor genannten Ausführungen ist es vorteilhaft, dass bei optisch aktiven cyclischen Estern der Formel I die in der zweiten Stufe durchgeführten Hydrolyse so lange durchgeführt wird, dass eine maximale Racemisierung von 10 %, bevorzugt maximal 5 %, besonders bevorzugt maximal 3 %, bezogen auf die Stereozentren des Rückstands erfolgt.

Die vorliegende Erfindung wird anhand der nachfolgenden Beispiele und Ausführungen näher erläutert, ohne die Erfindung auf die speziell dargestellten Parameter zu beschränken. Die beispielhaften Ausführungen betreffen insbesondere die Herstellung von Dilactid, sind jedoch ebenso auf andere cyclische Diester gemäß der allgemeinen Formel I übertragbar.

Erfindungsgemäß wird ein Verfahren zur Erhöhung der Ausbeute bei der Herstellung von Dilactid angeführt, bei dem in einem ersten Schritt die Depolymerisation von Milchsäure-Polykondensat bei einem Umsatz gestoppt wird, der so niedrig ist, dass sich ein niedriger, einstelliger Racemisierungsgrad (angegeben in % D-Isomere bezogen auf die Gesamtmenge Milchsäure) einstellt. Der dabei anfallende Rückstand wird mit Hilfe wässriger Milchsäure bei erhöhter Temperatur (ca. 95 °C) und leicht erniedrigtem Druck (900...950 mbar, zur Entfernung flüchtiger Bestandteile) hydrolysiert. Die Zusammensetzung des hydrolysierenden Mediums wird dabei so gewählt, dass das entstehende Hydrolysat exakt das gewünschte Molekulargewicht aufweist, um in eine entsprechende Prozessstufe zurückgeführt zu werden. Dem hydrolysierenden Medium werden unter Umständen Additive zugegeben, um Verunreinigungen zu binden. Gegebenenfalls wird anschließend durch Filtration, weitere Ausfällung oder Ionenaustausch die Menge der Verunreinigungen reduziert, um eine übermäßige Anreicherung im Prozess zu verhindern. Damit einhergehend wird eine geringe Menge des Hydrolysats als Reinigungsstrom (Purge) ausgeschleust. Der dabei entstehende Verlust an Milchsäure bezogen auf die insgesamt eingesetzte Milchsäure beträgt bevorzugt weniger als 0,5%. Das so gewonnene Polykondensat kann direkt in die Depolymerisation oder eine vorhergehende Polykondensationsstufe zurückgeführt werden, wo es mit dem frisch zugeführten Polykondensat vermischt wird. Dadurch wird zusätzlich die Energie zur Abdampfung des überschüssigen Wassers eingespart, was den Prozess wirtschaftlicher macht.

### Beispiele

### Beispiel 1

100 g eines Milchsäure-Polykondensats mit einem mittleren Molekulargewicht von 1000 g/mol werden in einem Ölbad nach Zugabe von 30 mg Sn(II)-Oktoat bei 20 mbar(a) auf 200 °C erhitzt. Die entstehende Dampfphase wird auskondensiert und enthält zu über 90 % Dilactid. Nach 3,5 Stunden wurden 95% des Polykondensats in Dilactid überführt. Der entstehende Rückstand hat ein mittleres Molekulargewicht von 3000 g/mol und geht bei 120 °C in den festen Zustand über.

Im Verlauf des Experiments werden mehrere Proben aus der entstehenden Dilactidphase entnommen. Die Bestimmung des Racemisierungsgrades X_{R} im Dilactid ergibt die in Tabelle 1 dargestellten Ergebnisse.

**Tabelle 1: Racemisierungsgrad X_{R} im Rückstand der zyklisierenden Depolymerisation.**

| | | | | | | | |
|---|---|---|---|---|---|---|---|
| Zeit [h] | 0 | 1 | 1.5 | 2 | 2.5 | 3 | 3.5 |
| X_{R} [% D] | 0.2 | 2.0 | 2.2 | 2.3 | 2.5 | 4.0 | 8.1 |

### Beispiel 2

100 g eines Milchsäure-Polykondensats mit einem mittleren Molekulargewicht von 1000 g/mol werden in einem Ölbad nach Zugabe von 30 mg Sn(II)-Oktoat bei 20 mbar(a) auf 200 °C erhitzt. Die entstehende Dampfphase wird auskondensiert und enthält zu über 90% Dilactid. Die Reaktion wird nach etwa 2.5 Stunden bei 80% Umsatz durch Brechen des Vakuums und Abkühlung auf Raumtemperatur gestoppt. Der entstehende Rückstand hat ein mittleres Molekulargewicht von 2000 g/mol und geht bei 115 °C in den festen Zustand über. Der Racemisierungsgrad im Rückstand liegt bei 2,5 % D-Einheiten.

### Beispiel 3

Jeweils 100 g des Rückstandes aus Beispiel 1 und Beispiel 2 werden aufgeschmolzen und in 100 g Hydrolysemedium, einer wässrigen Milchsäure-Lösung mit 50 wt-% Milchsäureanteil, bei 100 °C und Atmosphärendruck für 4 Stunden unter Rückfluss erhitzt. Die entstehende Lösung wird filtriert, um Feststoffpartikel zu entfernen. Die so erhaltene, homogene Lösung besitzt einen Milchsäureanteil von 88 wt-% (Rückstand 1) bzw. 87 wt% (Rückstand 2).

Der Racemisierungsgrad beträgt 8% (Rückstand 1) bzw. 3% (Rückstand 2).

### Beispiel 4

100 g des Rückstandes aus Beispiel 2 werden aufgeschmolzen und mit 50 g Hydrolysemedium, einer wässrigen Milchsäure-Lösung mit 60 wt-% Milchsäureanteil, bei 100 °C und Atmosphärendruck für 4 Stunden unter Rückfluss erhitzt. Die entstehende Lösung wird filtriert, um Feststoffpartikel zu entfernen. Die so erhaltene, homogene Lösung besitzt einen Milchsäureanteil von 92 wt-% (bestimmt über Titration).

### Beispiel 5

100 g des Rückstandes aus Beispiel 2 werden aufgeschmolzen und mit 50 g Hydrolysemedium, einer wässrigen Milchsäure-Lösung mit 95 wt-% Milchsäureanteil, bei 100 °C und Atmosphärendruck für 4 Stunden unter Rückfluss erhitzt. Die entstehende Lösung wird filtriert, um Feststoffpartikel zu entfernen. Die so erhaltene, homogene Lösung besitzt einen Milchsäureanteil größer 99 wt-%. Das mittlere Molekulargewicht der Lösung beträgt 300 g/mol.

### Beispiel 6

30 L eines Hydrolysats (100 g/L Milchsäure) werden unter Zugabe von 1,5 g/L Natriumsulfid (Na₂S) bei 25 °C und 50 rpm für 30 min lang gerührt. Der Niederschlag wird durch Filtration entfernt (Tabelle 2). Das Filtrat wird für weitere Aufarbeitung verwendet.

**Tabelle 2: Zinn im Filtrat des Hydrolysats vor und nach Filtration.**

| | **Zinn (mg/L)** | **Milchsäure (g/L)** |
|---|---|---|
| **Vor Zugabe von Na₂S** | 420 | 99,7 |
| **Nach Zugabe von Na₂S** | <2 | 99,4 |

### Beispiel 7

30 L eines Hydrolysats (10% Milchsäure) werden unter Zugabe von 1 g/L Thioacetamid (C₂H₅NS) bei 60°C und 50 rpm für 30 min lang gerührt. Der Niederschlag wird durch Zentrifugation entfernt (Tabelle 3). Das Filtrat wird für weitere Aufarbeitung verwendet.

**Tabelle 3: Zinn im Filtrat des Hydrolysats vor und nach Zentrifugation.**

| | **Zinn (mg/L)** | **Milchsäure (g/L)** |
|---|---|---|
| **Vor Zugabe von C₂H₅NS** | 420 | 99,7 |
| **Nach Zugabe von C₂H₅NS** | <2 | 99,6 |

### Beispiel 8

Das Hydrolysat aus Beispiel 5 wird entsprechend Beispiel 2 zu Dilactid umgesetzt. Dabei ergibt sich bei gleichen Versuchsbedingungen (Temperatur, Druck, Katalysatormenge) nach einer Reaktionszeit von 4 h ein Umsatz (Dilactid bezogen auf die eingesetzte Menge Polykondensat) von mindestens 90%.

### Beispiel 9

Das Hydrolysat aus Beispiel 6 oder 7 wird mit einem Milchsäure-olykondensat mit einem mittleren Molekulargewicht von 1000 g/mol gemischt, welches dem in Beispiel 1 eingesetzten Milchsäure-Polykondensat entspricht, so dass die entstehende Mischung ein Milchsäure-Polykondensat mit einem mittleren Molekulargewicht von 500 g/mol ergibt. Das entstandene Gemisch wird entsprechend Beispiel 1 zu Dilactid umgesetzt. Dabei ergibt sich nach 4 h ein Umsatz zu Dilactid von mindestens 90%.

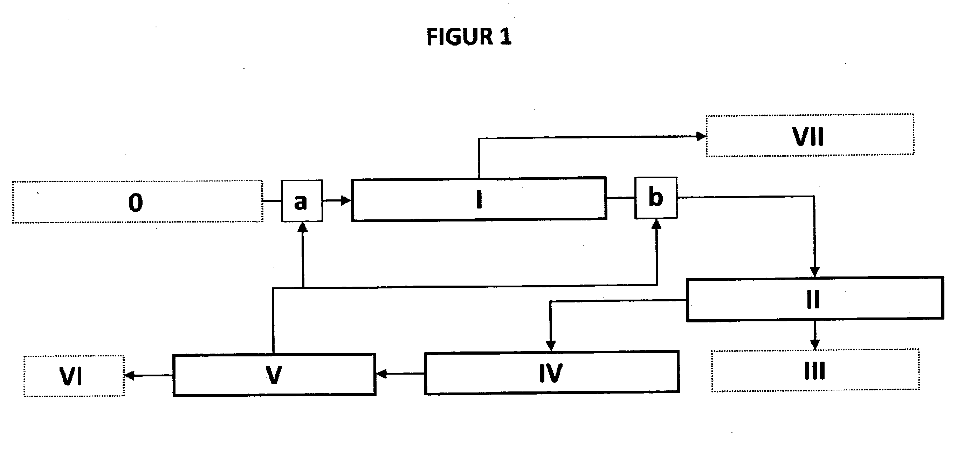

Figur 1 zeit ein Fließbild eines erfindungsgemäßen Verfahrens, das anhand des Beispiels der Herstellung von Dilactid erläutert wird. Die gleiche Verfahrensführung ist allerdings auch mit anderen cyclischen Diestern, wie beispielsweise Glycolid etc. durchführbar. Milchsäure wird aus einem Reservoir 0 über eine Weiche, ein T-Stück oder eine Pumpe a, einem Polykondensationsreaktor I hinzugefügt. Hierin erfolgt eine Polykondensation dieser Milchsäure zur oligomeren Milchsäuren. Daneben können ggf. im Reaktionsgemisch bereits oligomere Milchsäuren vorhanden sein. Bei der Polykondensation wird Wasser VII abgespalten, das beispielsweise dampfförmig und/oder unter vermindertem Druck entfernt werden kann. Das in der Stufe I erhaltene Polykondensat, das im wesentlichen aus oligomeren Milchsäuren besteht, wird einem Depolymerisationsreaktor II über eine Weiche, ein T-Stück oder eine Pumpe b zugeführt. Im Polymerisationsreaktor wird eine cyclisierende Depolymerisation durchgeführt, wobei Roh-Dilactid III entsteht, das aus dem Depolymeri-sationsreaktor II abgetrennt wird. Der im Depolymerisationsreaktor nach der cyclisierenden Depolymerisation verbleibende Rückstand entspricht im Wesentlichen oligomeren Dicarbonsäuren, die allerdings einen höheren Polykondensationsgrad, wie die aus der Stufe I erhaltenen oligomeren Dicarbonsäuren besitzen, aufweisen. Die bei der Depolymerisation zurückbleibenden oligomeren Dicarbonsäuren werden in eine Hydrolysestufe IV überführt, als hydrolisierendes Medium kann beispielsweise Wasser oder ein Gemisch aus Wasser und Milchsäure eingesetzt werden. Ggf. kann im Anschluss eine Aufbereitung V des Hydrolysats statt finden, wobei beispielsweise Feststoffe, insbesondere auch enthaltene Katalysatoren nach ggf. erfolgender Ausfällung abgetrennt werden können. Der bei der Aufbereitung abgetrennte Rückstand kann beispielsweise über einen Spülauslass (purge) VI abgetrennt werden. Das somit aufbereitete Hydrolysat kann beispielsweise über die Weichen, T-Stücke, oder Pumpen a und/oder b dem jeweiligen Fluss der einzelnen Verbindungen zugesetzt werden, sodass eine effektive Kreisführung der Komponenten möglich ist. Insbesondere ist bei diesem geschlossenen Kreislaufsystem vorteilhaft, dass lediglich Milchsäure aufgegeben wird und Roh-Dilactid mit hoher Ausbeute und hoher optischer Reinheit erhalten wird.

### Gesamtbeispiel

100 g eines Milchsäure-Polykondensats mit einem mittleren Molekulargewicht von 1000 g/mol werden in einem Laborkolben mit Rühreinheit in einem Ölbad nach Zugabe von 30 mg Sn(IV)-Oktoat bei 20 mbar(a) auf 200°C erhitzt. Die entstehende Dampfphase wird auskondensiert und enthält zu über 90 % Dilactid. Nach 2,5 Stunden wurden 80% des Polykondensats in Dilactid überführt. Der entstehende Rückstand hat ein mittleres Molekulargewicht von 2500 g/mol und geht bei 120 °C in den festen Zustand über, der Racemisierungsgrad in der Dilactid-Phase beträgt 2,5%.

100g des so erhaltenen Rückstands werden mit 50 g Hydrolysemedium (90%ige Milchsäure in wässriger Lösung) bei 100 °C und Atmosphärendruck für 4 Stunden unter Rückfluss und kontinuierlicher Durchmischung erhitzt. Die entstehende Lösung wird mit Natriumsulfid versetzt, und nach 30 min Durchmischung filtriert, um Feststoffpartikel zu entfernen. Die so erhaltene, homogene Lösung besitzt einen Milchsäureanteil von über 99 wt-% und einen Racemisierungsgrad von 2,5% und kann mit Polykondensat gemischt werden, um es direkt der Depolymerisationseinheit zuzuführen. Der Gesamtracemisierungsgrad des nach mehreren Zyklen erhaltenen Dilactids steigt trotz der Rückführung nie über den Maximalwert, der bei der direkten Depolymerisation bis zu einem Umsatz von 95% erreicht wird (siehe Beispiel 1). Die Gesamtausbeute wird bestimmt durch die Menge, die während der Hydrolyse durch Abtrennung der Verunreinigungen entfernt wird und liegt vorzugsgemäß bei über 99%, auf jeden Fall aber bei mehr als 95%.

## Patentansprüche

1. Verfahren zur Herstellung von cyclischen Estern der allgemeinen Formel I 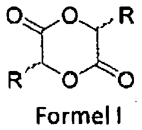 bei dem
a) in einer ersten Stufe ein Teil eines Gemischs oligomerer Carbonsäuren der Formel II 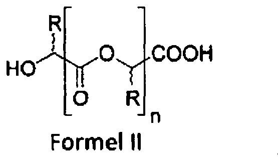 wobei in den Formeln I bis II jeweils R ausgewählt ist aus linearen oder verzweigten aliphatischen Resten mit 1 bis 6 Kohlenstoffatomen und in Formel II der gemittelte Wert für n zwischen 2 und 40 liegt,
durch cyclisierende Depolymerisation in den cyclischen Diester der allgemeinen Formel I überführt und der dabei entstehende cyclische Diester der allgemeinen Formel I abgetrennt wird, wobei der nicht in den cyclischen Diester der allgemeinen Formel I überführte Teil der oligomeren Carbonsäure der Formel II während der cyclisierenden Depolymerisation zu einem Rückstand abreagiert, der ein Gemisch oligomerer Carbonsäuren der Formel II mit einem höheren gemittelten Wert für n als das in die erste Stufe aufgegebene Gemisch oligomerer Carbonsäuren der Formel II beinhaltet,
b) in einer zweiten Stufe der in der ersten Stufe erhaltene Rückstand zu einem Gemisch oligomerer Carbonsäuren der Formel II, wobei der gemittelte Wert für n zwischen 1 und 20 liegt, zu einer alpha-Hydroxycarbonsäure der Formel III 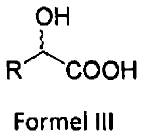 oder einem Gemisch aus oligomeren Carbonsäuren der Formel II mit einem gemittelten Wert für n zwischen 1 und 20 und einer alpha-Hydroxycarbonsäure der Formel III hydrolysiert wird, und
c) das in der zweiten Stufe erhaltene Hydrolysat zumindest teilweise oder vollständig in die erste Stufe aufgegeben wird,
**dadurch gekennzeichnet,**
**dass** die cyclisierende Depolymerisation der ersten Stufe solange durchgeführt wird, bis maximal 98 Gew.-% des eingesetzten Gemischs oligomerer Carbonsäuren der Formel II durch die cyclisierende Depolymerisation in den cyclischen Diester der allgemeinen Formel I überführt sind.

2. Verfahren nach Anspruch 1, **dadurch gekennzeichnet, dass** das in die erste Stufe aufgegebene Gemisch oligomerer Carbonsäuren der Formel II durch eine vorgeschaltete Polykondensation einer alpha-Hydroxycarbonsäure der Formel III 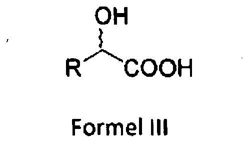 erhalten wird, wobei R die im Anspruch 1 angegebene Bedeutung hat.

3. Verfahren nach vorhergehendem Anspruch, **dadurch gekennzeichnet, dass** alternativ oder zusätzlich zur Aufgabe des Hydrolysats in die erste Stufe das Hydrolysat teilweise oder vollständig in die vorgeschaltete Polykondensation der alpha-Hydroxycarbonsäure der Formel III aufgegeben wird.

4. Verfahren nach einem der vorhergehenden Ansprüche, **dadurch gekennzeichnet, dass** die cyclisierende Depolymerisation der ersten Stufe solange durchgeführt wird, bis maximal 95 Gew.-%, weiter bevorzugt von 50 bis 95 Gew.-%, besonders bevorzugt 60 bis 85 Gew.-% des eingesetzten Gemischs oligomerer Carbonsäuren der Formel II durch die cyclisierende Depolymerisation in den cyclischen Diester der allgemeinen Formel I überführt sind.

5. Verfahren nach einem der vorhergehenden Ansprüche, **dadurch gekennzeichnet, dass** die in der zweiten Stufe durchgeführte partielle Hydrolyse des Rückstandes durch Umsetzung des Rückstandes mit einem hydrolysierenden Medium, ausgewählt aus der Gruppe bestehend aus Wasser, einer alpha-Hydroxycarbonsäure der Formel III oder Gemischen aus Wasser und einer alpha-Hydroxycarbonsäure der Formel III mit einem bevorzugten Wassergehalt von 1 bis 99 Gew.-%, bevorzugt 10 bis 80 %, besonders bevorzugt 20 bis 60 % durchgeführt wird.

6. Verfahren nach einem der vorhergehenden Ansprüche, **dadurch gekennzeichnet, dass** die in der zweiten Stufe durchgeführte partielle Hydrolyse bei Drücken zwischen 500 mbar und 2 bar, bevorzugt bei 900 mbar bis 1100 mbar und/oder bei Temperaturen zwischen 50 und 300 °C, bevorzugt zwischen 80 und 120 °C durchgeführt wird.

7. Verfahren nach einem der vorhergehenden Ansprüche, dass das Hydrolysat vor Rückführung aufgereinigt wird, insbesondere durch Filtration, Nanofiltration, Zentrifugation, Destillation, Ausfällung und/oder Abtrennung eines ggfs. vorhandenen Katalysators, lonentauschverfahren oder mehrerer der zuvor genannten Reinigungsverfahren.

8. Verfahren nach einem der vorhergehenden Ansprüche, **dadurch gekennzeichnet, dass** in der ersten Stufe ein Katalysator, bevorzugt ein Zinn-enthaltender Katalysator, insbesondere Zinn-(IV)-Octoat eingesetzt wird.

9. Verfahren nach einem der vorhergehenden Ansprüche, **dadurch gekennzeichnet, dass** die in der ersten Stufe durchgeführte cyclische Depolymerisation bei Temperaturen zwischen 100 und 300 °C, bevorzugt zwischen 150 und 250 °C durchgeführt wird.

10. Verfahren nach einem der vorhergehenden Ansprüche, **dadurch gekennzeichnet, dass** die cyclische Depolymerisation und/oder Abtrennung des in der ersten Stufe entstehenden cyclischen Diesters der allgemeinen Formel I bei gegenüber Normalbedingungen reduzierten Drücken, bevorzugt bei Drücken zwischen 0,1 und 500 mbar, weiter bevorzugt zwischen 10 und 100 mbar durchgeführt wird.

11. Verfahren nach einem der vorhergehenden Ansprüche, **dadurch gekennzeichnet, dass** die Stereozentren in Formel II zu mindestens 95 %, bevorzugt zu mindestens 98 %, besonders bevorzugt zu mindestens 99 % *S*-Konfiguration oder *R*-Konfiguration besitzen.

12. Verfahren nach vorhergehendem Anspruch, **dadurch gekennzeichnet, dass**
a) die Stereozentren in Formel II zu mindestens 95 %, bevorzugt zu mindestens 98 %, besonders bevorzugt zu mindestens 99 % *S-*Konfiguration besitzen und die cyclisierende Depolymerisation der ersten Stufe solange durchgeführt wird, bis maximal 10 %, bevorzugt maximal 5 %, besonders bevorzugt maximal 3 % des abgetrennten cyclischen Diesters der allgemeinen Formel I *R,S-*Konfiguration und/oder *R,R*-Konfiguration aufweisen, oder
b) die Stereozentren in Formel II zu mindestens 95 %, bevorzugt zu mindestens 98 %, besonders bevorzugt zu mindestens 99 % *R-*Konfiguration besitzen und die cyclisierende Depolymerisation der ersten Stufe solange durchgeführt wird, bis maximal 10 %, bevorzugt maximal 5 %, besonders bevorzugt maximal 3 % des abgetrennten cyclischen Diesters der allgemeinen Formel I *R,S-*Konfiguration und/oder S,S-Konfiguration aufweisen.

13. Verfahren nach einem der beiden vorhergehenden Ansprüche, **dadurch gekennzeichnet, dass** die in der zweiten Stufe durchgeführten Hydrolyse so lange durchgeführt wird, dass eine maximale Racemisierung von 10 %, bevorzugt maximal 5 %, besonders bevorzugt maximal 3 %, bezogen auf die Stereozentren des Rückstands erfolgt.

14. Verfahren nach einem der vorhergehenden Ansprüche, **dadurch gekennzeichnet, dass** R in den Formeln I bis III Methyl ist.

## Claims

1. Method for the production of cyclic esters of the general formula I 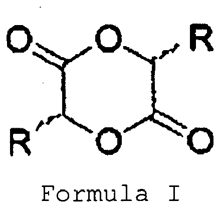 in which
a) in a first step, a part of a mixture of oligomer carboxylic acids of formula II 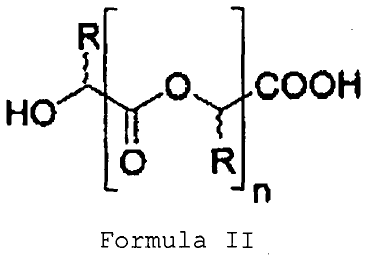 wherein, in formulae I to II, each R is selected from linear or branched aliphatic radicals having 1 to 6 carbon atoms and, in formula II, the average value for n is between 2 and 40,
is converted into the cyclic diester of the general formula I using cyclising depolymerisation and the thus arising cyclic diester of the general formula I is separated, wherein the part of the oligomer carboxylic acid of formula II which is not converted into the cyclic diester of the general formula I reacts into a residue during the cyclising depolymerisation, said residue containing a mixture of oligomer carboxylic acids of formula II having a higher average value for n than the mixture of oligomer carboxylic acids of formula II used in the first step,
b) in a second step, the residue obtained in the first step is hydrolysed into a mixture of oligomer carboxylic acids of formula II, wherein the average value for n is between 1 and 20, into an alpha-hydroxy carboxylic acid of formula III 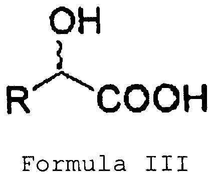 or a mixture of oligomer carboxylic acids of formula II with an average value for n of between 1 and 20 and an alpha-hydroxy carboxylic acid of formula III, and
c) the hydrolysate obtained in the second step is used at least partially or completely in the first step,
**characterised in that**,
the cyclising depolymerisation of the first step is carried out until a maximum of 98% b.w. of the used mixture of oligomer carboxylic acids of formula II is converted into the cyclic diester of general formula I using cyclising depolymerisation.

2. Method according to claim 1, **characterised in that** the mixture of oligomer carboxylic acids of formula II which is used in the first step is obtained by an upstream polycondensation of an alpha-hydroxy carboxylic acid of formula III 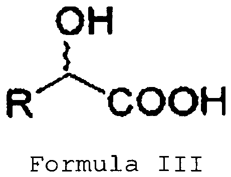 wherein R has the meaning specified in claim 1.

3. Method according to the preceding claim, **characterised in that**, alternatively or additionally to the use of the hydrolysate in the first step, the hydrolysate is partially or completely used in the upstream polycondensation of the alpha-hydroxy carboxylic acid of formula III.

4. Method according to one of the preceding claims, **characterised in that** the cyclic depolymerisation of the first step is carried out until a maximum of 95% by weight, more preferably 50 to 95% by weight, particularly preferably 60 to 85% by weight of the used mixture of oligomer carboxylic acids of formula II are converted into the cyclic diester of the general formula I by the cyclising depolymerisation.

5. Method according to one of the preceding claims, **characterised in that** the partial hydrolysis of the residue carried out in the second step is carried out by conversion of the residue with a hydrolysing medium, selected from the group consisting of water, an alpha-hydroxy carboxylic acid of formula III or mixtures of water and an alpha-hydroxy carboxylic acid of formula III having a preferred water content of 1 to 99% by weight, preferably 10 to 80%, particularly preferably 20 to 60%.

6. Method according to one of the preceding claims, **characterised in that** the partial hydrolysis carried out in the second step is carried out at pressures of between 500 mbar and 2 bar, preferably at 900 mbar to 1100 mbar and/or at temperatures of between 50 and 300°C, preferably between 80 and 120°C.

7. Method according to one of the preceding claims, **characterised in that** the hydrolysate is purified before recirculation, in particular by filtration, nano-filtration, centrifugation, distillation, precipitation and/or separation of a potentially present catalyst, ion exchange method or several of the previously referred to purification methods.

8. Method according to one of the preceding claims, **characterised in that**, in the first step, a catalyst, preferably a tin-containing catalyst, in particular tin(IV) octoate, is used.

9. Method according to one of the preceding claims, **characterised in that** the cyclic depolymerisation carried out in the first step is carried out at temperatures of between 100 and 300°C, preferably between 150 and 250°C.

10. Method according to one of the preceding claims, **characterised in that** the cyclic depolymerisation and/or separation of the cyclic diester of the general formula I arising in the first step is carried out at reduced pressures compared to normal conditions, preferably at pressures of between 0.1 and 500 mbar, more preferably between 10 and 100 mbar.

11. Method according to one of the preceding claims, **characterised in that** the stereocentres in formula II have an at least 95%, preferably at least 98%, particularly preferably at least 99% S-configuration or R-configuration.

12. Method according to the preceding claim, **characterised in that**
a) the stereocentres in formula II have an at least 95%, preferably at least 98%, particularly preferably at least 99% S-configuration and the cyclising depolymerisation of the first step is carried out until a maximum of 10%, preferably a maximum of 5%, particularly preferably a maximum of 3% of the separated cyclic diester of the general formula I have R,S configuration and/or R,R configuration, or
b) the stereocentres in formula II have an at least 95%, preferably at least 98%, particularly preferably at least 99% R-configuration and the cyclising depolymerisation of the first step is carried out until a maximum of 10%, preferably a maximum of 5%, particularly preferably a maximum of 3% of the separated cyclic diester of general formula I have R,S configuration and/or S,S configuration.

13. Method according to one of the two preceding claims, **characterised in that** the hydrolysis carried out in the second step is carried out until a maximum racemisation of 10%, preferably a maximum of 5%, particularly preferably a maximum of 3%, occurs with respect to the stereocentres of the residue.

14. Method according to one of the preceding claims, **characterised in that** R is methyl in formulae I to III.

## Revendications

1. Procédé de fabrication d'esters cycliques de formule générale I 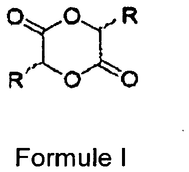 selon lequel
a) lors d'une première étape, une partie d'un mélange d'acides carboxyliques
oligomères de formule II 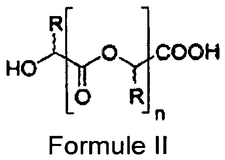 dans les formules I à II, chaque R étant choisi parmi les radicaux aliphatiques linéaires ou ramifiés de 1 à 6 atomes de carbone et, dans la formule II, la valeur moyennée pour n étant comprise entre 2 et 40,
est transformée par dépolymérisation cyclisante en le diester cyclique de formule générale I et le diester cyclique de formule générale I formé est séparé, la partie des acides carboxyliques oligomères de formule II non transformée en les diesters cycliques de formule générale I réagissant pendant la dépolymérisation cyclisante pour former un résidu qui contient un mélange d'acides carboxyliques oligomères de formule II ayant une valeur moyennée plus élevée pour n que le mélange d'acides carboxyliques oligomères de formule II introduit dans la première étape,
b) lors d'une seconde étape, le résidu obtenu lors de la première étape est hydrolysé en un mélange d'acides carboxyliques oligomères de formule II, la valeur moyennée pour n étant comprise entre 1 et 20, en un acide alpha-hydroxycarboxylique de formule III 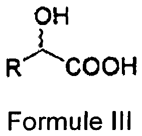 ou un mélange d'acides carboxyliques oligomères de formule II ayant une valeur moyennée pour n comprise entre 1 et 20 et un acide alpha-hydroxycarboxylique de formule III, et
c) l'hydrolysat obtenu lors de la seconde étape est introduit au moins en partie voire en totalité dans la première étape,
**caractérisé en ce que**
la dépolymérisation cyclisante de la première étape est réalisée jusqu'à ce qu'au plus 98 % en poids du mélange d'acides carboxyliques oligomères de formule II utilisé soit transformé par la dépolymérisation cyclisante en le diester cyclique de formule générale I.

2. Procédé selon la revendication 1, **caractérisé en ce que** le mélange d'acides carboxyliques oligomères de formule II introduit dans la première étape, est obtenu par une polycondensation en amont d'un acide alpha-hydroxycarboxylique de formule III 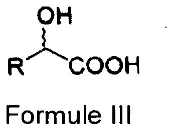 R ayant la signification indiquée dans la revendication 1.

3. Procédé selon la revendication précédente, **caractérisé en ce qu'**en variante ou en plus de l'introduction de l'hydrolysat dans la première étape, l'hydrolysat est introduit en partie voire en totalité dans la polycondensation en amont de l'acide alpha-hydroxycarboxylique de formule III.

4. Procédé selon l'une quelconque des revendications précédentes, **caractérisé en ce que** la dépolymérisation cyclisante de la première étape est réalisée jusqu'à ce qu'au plus 95 % en poids, de manière davantage préférée de 50 à 95 % en poids, de manière particulièrement préférée de 60 à 85 % en poids, du mélange d'acides carboxyliques oligomères de formule II utilisé soit transformé par la dépolymérisation cyclisante en le diester cyclique de formule générale I.

5. Procédé selon l'une quelconque des revendications précédentes, **caractérisé en ce que** l'hydrolyse partielle du résidu réalisée lors de la seconde étape est réalisée par mise en réaction du résidu avec un milieu hydrolysant, choisi dans le groupe constitué par l'eau, un acide alpha-hydroxycarboxylique de formule III ou des mélanges d'eau et d'un acide alpha-hydroxycarboxylique de formule III ayant une teneur en eau préférée de 1 à 99 % en poids, de préférence de 10 à 80 %, de manière particulièrement préférée allant de 20 à 60 %.

6. Procédé selon l'une quelconque des revendications précédentes, **caractérisé en ce que** l'hydrolyse partielle réalisée lors de la seconde étape est réalisée à des pressions comprises entre 500 mbar et 2 bar, de préférence de 900 mbar à 1 100 mbar, et/ou à des températures comprises entre 50 et 300 °C, de préférence entre 80 et 120 °C.

7. Procédé selon l'une quelconque des revendications précédentes, **caractérisé en ce que** l'hydrolysat est purifié avant le recyclage, notamment par filtration, nanofiltration, centrifugation, distillation, précipitation et/ou séparation d'un catalyseur éventuellement présent, des procédés d'échange d'ions ou plusieurs des procédés de purification susmentionnés.

8. Procédé selon l'une quelconque des revendications précédentes, **caractérisé en ce qu'**un catalyseur, de préférence un catalyseur contenant de l'étain, notamment de l'octoate d'étain (IV), est utilisé lors de la première étape.

9. Procédé selon l'une quelconque des revendications précédentes, **caractérisé en ce que** la dépolymérisation cyclique réalisée lors de la première étape est réalisée à des températures comprises entre 100 et 300 °C, de préférence entre 150 et 250 °C.

10. Procédé selon l'une quelconque des revendications précédentes, **caractérisé en ce que** la dépolymérisation cyclique et/ou la séparation du diester cyclique de formule générale I formé lors de la première étape est réalisée à des pressions réduites par rapport aux conditions normales, de préférence à des pressions comprises entre 0,1 et 500 mbar, de manière davantage préférée entre 10 et 100 mbar.

11. Procédé selon l'une quelconque des revendications précédentes, **caractérisé en ce que** les stéréocentres dans la formule II présentent à au moins 95 %, de préférence à au moins 98 %, de manière particulièrement préférée à au moins 99 %, la configuration S ou la configuration R.

12. Procédé selon la revendication précédente, **caractérisé en ce que**
a) les stéréocentres dans la formule II présentent à au moins 95 %, de préférence à au moins 98 %, de manière particulièrement préférée à au moins 99 %, la configuration S, et la dépolymérisation cyclisante de la première étape est réalisée jusqu'à ce qu'au plus 10 %, de préférence au plus 5 %, de manière particulièrement préférée au plus 3 %, du diester cyclique de formule générale I séparé présente la configuration R,S et/ou la configuration R,R, ou
b) les stéréocentres dans la formule II présentent à au moins 95 %, de préférence à au moins 98 %, de manière particulièrement préférée à au moins 99 %, la configuration R, et la dépolymérisation cyclisante de la première étape est réalisée jusqu'à ce qu'au plus 10 %, de préférence au plus 5 %, de manière particulièrement préférée au plus 3 %, du diester cyclique de formule générale I séparé présente la configuration R,S et/ou la configuration S,S.

13. Procédé selon l'une quelconque des deux revendications précédentes, **caractérisé en ce que** l'hydrolyse réalisée lors de la seconde étape est réalisée jusqu'à ce qu'une racémisation maximale de 10 %, de préférence d'au plus 5 %, de manière particulièrement préférée d'au plus 3 %, par rapport aux stéréocentres du résidu, ait lieu.

14. Procédé selon l'une quelconque des revendications précédentes, **caractérisé en ce que** R dans les formules I à III représente le méthyle.
